# DEMANDE DE BREVET EUROPEEN

(11) **EP 0 864 296 A1**
(43) Date de publication de la demande: **16.09.1998**
(21) Numéro de dépôt: 98400572.8
(22) Date de dépôt: 11.03.1998
(51) Int. Cl.: A61B 10/00

(54) **Dispositif amélioré de prélèvement utérin**

(30) Priorité: 11.03.1997 FR 9702840
(71) Demandeur: LABORATOIRE C.C.D., 75008 Paris (FR)
(72) Inventeur: Choay, Patrick, 75007 Paris (FR)
(74) Mandataire: Wagret, Frédéric

(57) **Abrégé**

La présente invention concerne un dispositif de prélèvement utérin du type comprenant un manche longiligne (1) associé à l'une de ses extrémités (9) à au moins une tige longitudinale (6) sensiblement cylindrique, et sur laquelle sont disposées transversalement une pluralité d'éléments allongés (11) aptes à gratter la muqueuse utérine et à prélever des cellules, caractérisé en ce que la tige longitudinale (6) et les éléments (11) sont réalisés à l'aide de deux matériaux thermoplastiques respectifs, et en ce que la matériau formant lesdits éléments (11) présente une rigidité, dans le sens transversal, inférieure au matériau formant ladite tige longitudinale (6).

## Description

La présente invention concerne un dispositif destiné au prélèvement de cellules ou de muqueuses de l'endocol et/ou de l'exocol utérin, en vue d'un examen cytologique des parties prélevées.

On connaît l'importance de ces examens qui permettent le suivi et le dépistage de maladies et d'infections de l'appareil génital féminin.

Les opérations de prélèvement sont réalisées, de manière connue, à l'aide d'outil à main, comportant un manche longiligne, et à une extrémité duquel est prévu un organe de prélèvement.

Compte tenu de la forme du col de l'utérus, on distingue deux zones où sont effectués les prélèvements, à savoir une zone sensiblement cylindrique, appelée endocol, formant le corps de l'utérus, et une seconde zone, à l'entrée de l'utérus, en forme de demi tore, appelé exocol.

Ainsi, la paroi de l'endocol est sensiblement alignée avec la cavité vaginale par où est introduit l'outil, tandis que la paroi de l'exocol est sensiblement transversale à cette direction.

Ainsi, on comprend que le prélèvement dans ces deux régions doit s'effectuer de deux manières différentes compte tenu de leur direction sensiblement orthogonale respective.

Ces deux types de prélèvement font appel à des organes de grattage présentant des caractéristiques différentes, à l'aide de deux outils différents, ce qui présente un inconvénient majeur pour la patiente; celle-ci doit supporter deux opérations successives, avec la gêne en résultant, compte tenu de l'allongement dans le temps de cette opération. Ceci est d'autant plus vrai que le praticien doit alors, après le premier prélèvement, placer en sûreté les parties prélevées dans un liquide physiologique ou autre, ce qui allonge d'autant l'opération de prélèvement pour la patiente, avant de pouvoir utiliser un autre outil et recommencer. Du point de vue économique, la réalisation de deux outils augmente les coûts, puisque ces dispositifs sont à usage unique.

On a tenté de remédier à cette situation, de manière connue, en prévoyant un outil dont le manche est pourvu à ses deux extrémités de deux moyens de prélèvement destinés respectivement à opérer sur l'endocol et l'exocol. Cette disposition, si elle permet de réduire les coûts, ne résout pas la difficulté consistant à effectuer deux opérations de prélèvement successives, avec les inconvénients qui en résultent pour la patiente, et également pour le praticien qui doit assurer la conservation physiologique du premier prélèvement réalisé sur une des extrémités de l'outil.

Toujours de manière connue, on a proposé de munir un outil à l'une de ses extrémités de deux organes distincts de prélèvement, le premier constitué d'éléments transversaux, sous forme de poils, destiné au prélèvement sur l'endocol, et d'un second organe de prélèvement transversal, sous la forme d'une spatule, destiné au prélèvement sur l'exocol.

Cette disposition, si elle permet de réaliser les deux prélèvements en une seule pénétration, est gênante, voire douloureuse pour la patiente compte tenu notamment du caractère relativement rigide la spatule destinée au prélèvement sur l'exocol, malgré toute la dextérité que peut posséder le praticien.

Un autre problème réside en la difficulté de prélever les muqueuses, de manière indolore, le plus rapidement possible, et dans des conditions optimales du point de vue de l'examen (notamment cytologique) ultérieur.

A cet égard, on connaît des dispositifs réalisés sous la forme d'un manche en tube creux, associé à l'une des ses extrémités à une tige métallique, et sur laquelle sont montés des éléments en plastique, formant une sorte de brosse.

Si les poils en plastique souple répondent parfaitement aux conditions de prélèvement optimales, la présence d'une tige métallique rend l'opération de prélèvement difficile et oblige le praticien à beaucoup de prudence, ce qui peut nuire à la qualité de prélèvement.

Du point de vue économique, cette forme de réalisation est très onéreuse par la présence de trois matériaux de type différent, et par la forme de réalisation de la tige métallique torsadée dans laquelle sont disposés les poils de la brosse.

Dans ce contexte, l'invention a pour but de remédier aux inconvénients ci-dessus, et propose un outil ou dispositif de prélèvement, qui permet de réaliser les opérations de prélèvement, à la fois dans l'endocol et l'exocol, dans des conditions optimales du point de vue de l'examen ultérieur, et également de manière à gêner le moins possible la patiente, dans des conditions de sécurité optimale, et permettant au praticien de réaliser ces opérations dans un minimum de temps et sans manipulation inutile, tout en présentant des conditions de fabrication simple et peu onéreuse.

A cette fin, selon l'invention, le dispositif de prélèvement utérin, du type comprenant un manche longiligne associé à l'une de ses extrémités à au moins une tige longitudinale sensiblement cylindrique et sur laquelle sont disposés transversalement une pluralité d'éléments allongés aptes à gratter la muqueuse utérine, et à prélever des cellules, est caractérisé en ce que la tige et les éléments sont réalisés en matériau thermoplastique, et en ce que le matériau formant lesdits éléments présente une rigidité, dans le sens transversal, inférieure à celle du matériau formant ladite tige.

De préférence, la tige solidaire est une embase, apte à coopérer avec l'extrémité du manche creux, formant manchon, et également la tige comporte un anneau de butée entre la tige et l'embase.

Selon une variante de réalisation, le manche et la tige sont réalisés en une seule pièce, venue de moulage.

A titre d'exemple, le matériau formant la tige et le manche est du polyuréthanne d'une dureté SHORE donnée, et le matériau formant les éléments de grattage est du polyuréthanne d'une dureté SHORE inférieure.

Les éléments de grattage sont disposés alignés de manière que la base de deux éléments contigus soit quasiment en contact. De préférence, les éléments sont alignés selon au moins une génératrice de la tige, et de préférence selon deux génératrices diamétralement opposées.

Afin de permettre un prélèvement au niveau de l'exocol, le dispositif comporte une seconde tige transversale, pourvue d'éléments de grattage, ladite tige transversale présentant de préférence un profil courbe.

Les éléments de grattage présentent une forme sensiblement tronconique, où selon une autre forme, ils présentent une section carrée ou rectangulaire.

La tige comporte un, deux ou plus, et de préférence trois, rangées d'éléments de grattage parallèles, chaque rangée étant parallèle à l'autre.

L'invention concerne également un procédé pour la réalisation d'un dispositif de prélèvement tel que mentionné ci-dessus, et caractérisé en ce qu'on réalise par moulage la tige dans un matériau présentant une rigidité propre à permettre l'usage du dispositif, et on rapporte, par moulage, sur ladite tige les éléments de grattage, en injectant un matériau présentant des caractéristiques de rigidité inférieure à celui formant la tige. Ensuite, on associe la tige et le manche par fixation de toute nature, soudure, colle, ou similaires. Le manche peut être, par exemple, un tube creux (en plastique) dans lequel on enfile à une extrémité, à force, l'extrémité inférieure de la tige portant les éléments de grattage.

Selon une première variante du procédé, la tige et les éléments sont réalisés simultanément par une opération de moulage dite à bi-injection, par injection simultanée de deux matériaux différents, présentant des caractéristiques de rigidité distinctes.

Selon une seconde variante, la tige et le manche sont réalisés par moulage en une seule pièce, puis les éléments de grattage sont rapportés par moulage.

L'invention sera bien comprise à la lumière de la description qui suit se rapportant à l'exemple illustratif et non limitatif, en regard du dessin annexé dans lequel :
- la figure 1 est une vue de côté montrant le dispositif, et plus particulièrement l'extrémité du manche pourvu des moyens de grattage;
- la figure 2 est une vue en perspective du dispositif de prélèvement, le manche n'étant pas représenté;
- La figure 3 est une vue de face du dispositif des figures 1 et 2;
- La figure 4 est une vue de dessus de ce même dispositif;
- Les figures 5A et 5B montrent, à une échelle agrandie, un élément de grattage, respectivement de côté et vu de dessus;
- Les figures 6, 7 et 8 montrent une autre forme de réalisation du dispositif de l'invention, respectivement de côté, de face et en perspective;
- La figure 9 montre une vue de dessus du dispositif des figures 6, 7 et 8; et
- La figure 10 montre, à une échelle agrandie, en vue de dessus, un élément de grattage.

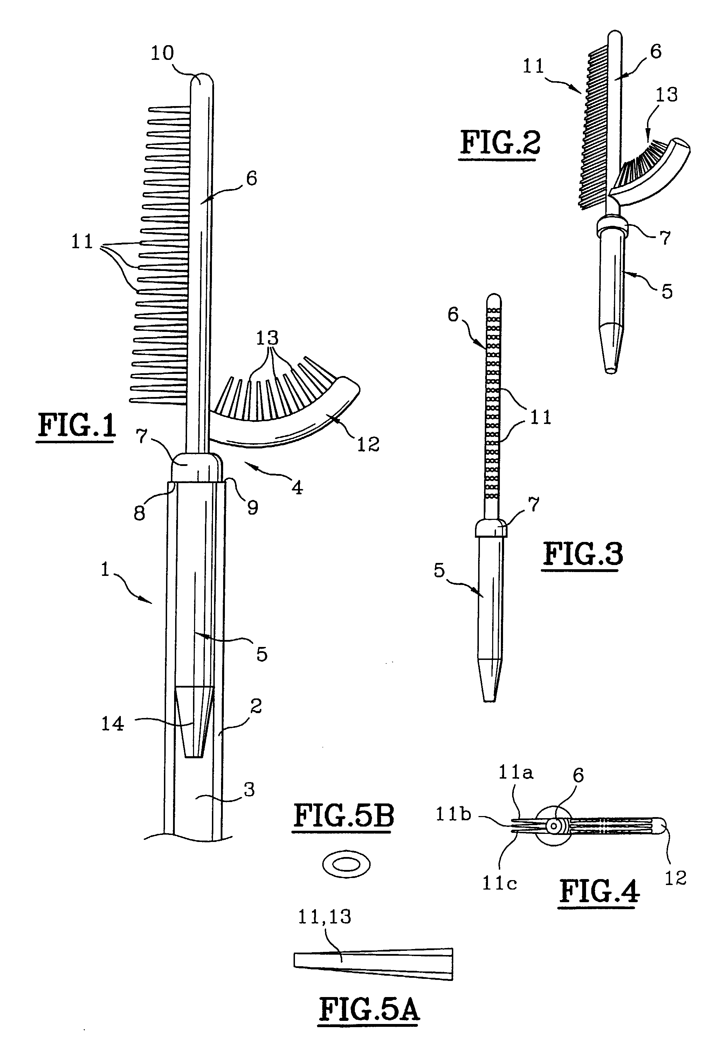

En référence à la figure 1, le dispositif comporte un manche référencé 1, et dont seule la partie d'extrémité active (opposée au praticien) est représentée sur la figure 1. Le manche 1 est constitué d'un tube creux en matériau plastique de préférence. La paroi 2 du manche creux 1 définit un espace intérieur 3, de forme cylindrique et apte à recevoir, du côté de l'extrémité du manche 1, un ensemble de prélèvement et de grattage, portant la référence générale 4, et comprenant d'une part une embase 5, et d'autre part une tige 6. L'embase 5 et la tige 6 sont colinéaires et également colinéaires à l'axe du manche 1, et sont également de forme générale cylindrique.

Le diamètre de l'embase correspond au diamètre intérieur du manche creux 1 de manière à ce que celui-ci puisse recevoir dans l'espace intérieur 3 ladite embase 5, avec un léger frottement, pour permettre le maintien de l'embase 5 dans le manche 1.

Entre la tige 6 et l'embase 5, est prévu un anneau de butée 7, formant un épaulement 8 contre lequel porte l'extrémité 9 du manche 1.

La tige 6 est de préférence de forme cylindrique à section circulaire et comporte une extrémité distale 10 arrondie. Sur la tige 6 est disposés un ensemble d'éléments de grattage, constituant les poils d'une brosse, et référencés 11.

Selon la forme de réalisation montrée sur la figure 1, le dispositif comporte en outre une seconde tige 12 transversale à la tige longitudinale 6, et présentant de préférence une forme générale courbe, dont la concavité est tournée vers l'extrémité distale 10 de la tige 6.

La tige transversale 12 comporte également des éléments de grattage et de prélèvement, formant les poils d'une sorte de brosse, et référencés 13. Afin de permettre la pénétration de l'embase 5 dans l'espace creux 3 du manche 1, l'extrémité de l'embase 5, du côté opposé à la tige 6, comprend une partie 14 tronconique.

La figure 2 montre en perspective le dispositif de prélèvement. Le manche 1 n'a pas été représenté, pour des raisons de clarté. On retrouve sur cette figure les mêmes éléments, à savoir l'embase 5, l'anneau de butée 7, la tige longitudinale 6, la tige transversale 12, respectivement pourvues chacune d'une série de poils de prélèvement 11 et 13. La figure 3 montre le dispositif de la figure 2, en vue de face, la tige transversale 12 étant perpendiculaire sensiblement au plan de la figure. La figure 4 montre le dispositif en vue de dessus.

On constate, dans la forme de réalisation des figures 1 à 4, que la première série de poils 11 prévue sur la tige longitudinale 6, forme trois rangées parallèles de poils, à savoir 11a, 11b et 11c, la rangée centrale 11b étant disposée selon une génératrice du cylindre formée par la tige 6. Les figures 3 et 4 montrent les trois rangées de poils de la première série, associées à la tige 6. D'une manière plus générale, la tige 6 comporte au moins une rangée, et de préférence deux ou trois, de poils parallèles, l'une des rangées étant disposée selon une génératrice de la tige correspondante.

Il en est de même de la deuxième série de poils 13 associée à la seconde tige transversale 12, comme montré sur la figure 4. Les poils 13 de la seconde série, étant fixés transversalement à la paroi de la tige 12, et celle-ci étant de forme générale courbe, les axes des poils de la seconde série 13 ne sont pas parallèles entre eux mais convergent. Leurs extrémités définissent une surface en arc de cercle, propre à faciliter le prélèvement au niveau de l'exocol.

Les figures 5A et 5B montrent respectivement en vue de côté et en vue de dessus un poil de la première ou de la seconde série 11 ou 13. Chaque poil présente une forme sensiblement tronconique, à base elliptique.

Selon une première forme de réalisation, le dispositif de l'invention est fabriqué de la manière suivante.

Dans un premier temps, on réalise par moulage l'ensemble comprenant l'embase 5, l'anneau de butée, et la tige 6.

Cet ensemble est réalisé en un matériau thermoformable ou thermoplastique, par moulage par injection, tel que par exemple du polyuréthanne. Ce matériau est choisi de manière à présenter une certaine rigidité, en toute hypothèse suffisante pour permettre l'utilisation du dispositif et sa tenue, lorsqu'il est introduit dans l'utérus de la patiente.

On entend par rigidité, la propriété du matériau à se plier dans le sens transversal, c'est-à-dire dans le sens perpendiculaire à l'axe longitudinal du manche ou de la direction d'opération (également parallèle à l'axe du vagin et de l'utérus).

Dans un second temps, on rapporte par moulage (par injection par exemple), sur les tiges respectives longitudinale 6 et transversale 12, la première série de poils 11 et la seconde série de poils 12, étant entendu que ces dernières sont réalisées en un matériau présentant une rigidité inférieure à celle constituant l'ensemble embase/tiges. Par exemple, le matériau constituant les poils peut être du polyuréthanne.

Dans un troisième temps, la tige est associée à un manche et fixée à celui-ci.

Selon une seconde forme de mise en oeuvre du procédé selon l'invention, on réalise l'ensemble du dispositif en une seule et même opération par moulage bi-injection, dans lequel on injecte dans un moule deux matériaux présentant des rigidités différentes, le premier matériau étant appelé à remplir l'espace du moule correspondant à l'embase 5, l'anneau 7 et les tiges 6 et 12, tandis que le second matériau de rigidité inférieure est appelé à remplir l'espace du moule correspondant aux première et seconde séries 11 et 13 de poils de grattage et prélèvement.

Selon une troisième forme de mise en oeuvre du procédé, on réalise la tige et le manche en une seule pièce, par moulage, puis on rapporte les éléments de grattage par moulage sur la tige.

Dans la forme de réalisation des figures 1 à 5, la forme des poils est avantageuse dans la mesure où elle permet d'accentuer l'effet de grattage et de prélèvement, grâce à la forme tronconique des poils, qui autorise ainsi une rigidité décroissante, en s'éloignant de la tige. On entend par rigidité la faculté de l'élément correspondant de se plier dans le sens transversal, c'est-à-dire perpendiculairement à sa direction longitudinale.

De plus, le fait de disposer les poils par séries parallèles, renforce l'effet de brosse obtenu par les poils. On augmente ainsi la quantité de muqueuse prélevée, sans nuire au confort de la patiente, et à la sécurité de l'opération.

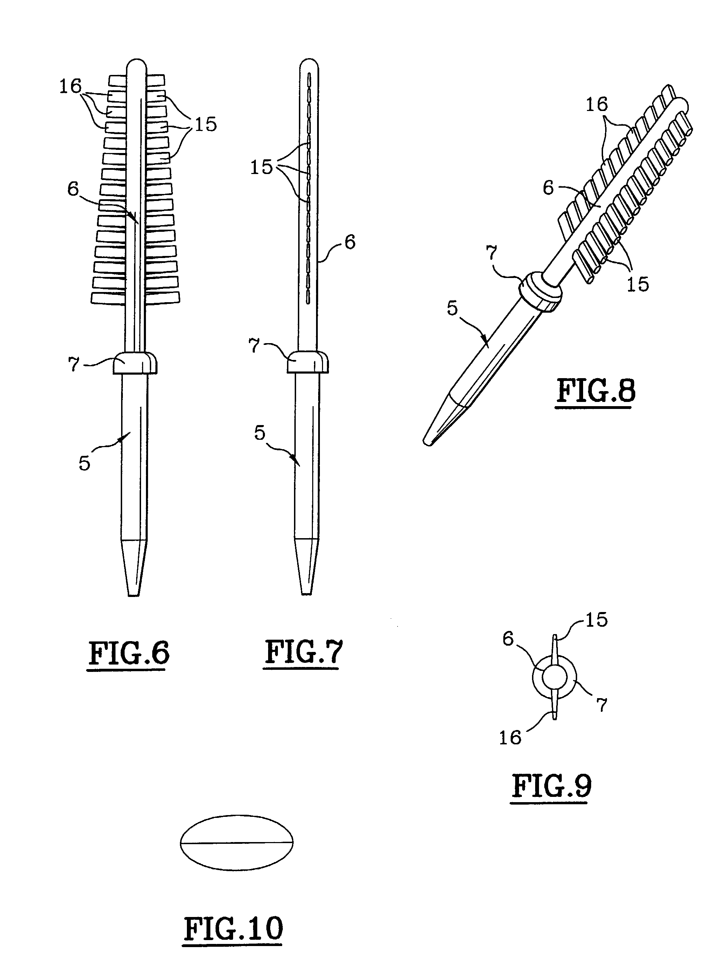

On décrit ci-après plus en détail une seconde forme de réalisation du dispositif de l'invention, en référence aux figures 6 à 10.

Dans cette forme de réalisation, le dispositif ne comporte que la seule tige longitudinale 6, la tige transversale 12 n'étant pas présente. Ce dispositif est donc destiné au prélèvement dans l'endocol.

Pour des raisons de commodité, les éléments ou organes identiques à ceux de la forme de réalisation des figures 1 à 5, portent les mêmes références. Ainsi, on retrouve l'embase 5, l'anneau de butée 7 et la tige longitudinale 6.

En référence aux figures 6 à 8, le dispositif comporte deux séries de poils formant brosse référencées 15 et 16, et alignées selon deux génératrices du cylindre formé par la tige longitudinale 6, et diamétralement opposées. La figure 9 montre plus particulièrement, en vue de dessus, la disposition des poils par rapport à la tige longitudinale 6.

Les poils, dans cette forme de réalisation, sont de préférence conformés en forme de cylindre à base ovale, ou d'ellipse.

Dans les formes de réalisation des figures, que ce soit les figures 1 à 5 ou les figures 6 à 10, les poils de chaque série sont à leur base sensiblement jointifs ou très proche les uns des autres, c'est-à-dire du côté de la tige correspondante (longitudinale 6 et/ou tige transversale 12).

La forme et la section des éléments de grattage peut être également de forme cylindrique, à base carrée, rectangulaire, trapézoïdale, ou tout autre polygone.

Enfin, selon une autre variante de l'invention, l'ensemble du manche 1, l'embase 5, l'anneau de butée 7 et la tige 6 (et éventuellement les éléments de grattage) sont réalisés en une seule pièce et une même opération de moulage. Ainsi, selon cette forme de réalisation, qui n'est pas représentée, le manche 1 et l'embase 5 sont venus d'une seule et même pièce. Selon cette forme de réalisation, le manche peut être plein et réalisé en un matériaux thermoformable venu d'une seule et même pièce avec la tige 6 longitudinale et la tige 12 transversale.

## Revendications

1. Dispositif de prélèvement utérin du type comprenant un manche longiligne (1) associé à l'une de ses extrémités (9) à au moins une tige longitudinale (6) sensiblement cylindrique, et sur laquelle sont disposés transversalement une pluralité d'éléments allongés (11) aptes à gratter la muqueuse utérine et à prélever des cellules, la tige longitudinale (6) et les éléments (11) étant réalisés à l'aide de matériau thermoplastique, caractérisé en ce que le matériau formant lesdits éléments (11) présente une rigidité, dans le sens transversal, inférieure au matériau formant ladite tige longitudinale (6).

2. Dispositif selon la revendication 1, caractérisé en ce que le matériau formant la tige longitudinale (6) est du polyuréthanne et en ce que le matériau formant les éléments allongés aptes à gratter la muqueuse (11,13,15,16) est du polyuréthanne.

3. Dispositif selon la revendication 1, caractérisé en ce que lesdits éléments (11,13,15,16) sont alignés selon des génératrices diamétralement opposées.

4. Dispositif selon l'une des revendications 1 à 3, caractérisé en ce qu'il comporte une seconde tige (12) disposée transversalement par rapport au manche et pourvue d'éléments de grattage (13).

5. Dispositif selon la revendication 4, caractérisé en ce que ladite seconde tige transversale (12) présente un profil courbe de concavité tournée vers l'extrémité distale du dispositif.

6. Dispositif selon l'une des revendications 1 à 5, caractérisé en ce que les éléments allongés aptes à gratter la muqueuse (11,13) présentent une forme sensiblement tronconique.

7. Dispositif selon l'une des revendications 1 à 6, caractérisé en ce que les éléments allongés aptes à gratter la muqueuse (15,16) présentent une section cylindrique à base elliptique, carrée ou rectangulaire.

8. Dispositif selon l'une des revendications 1 à 7, caractérisé en ce que la tige longitudinale (6) comporte au moins une rangée, et de préférence deux ou trois, d'éléments (11,13) parallèles allongés aptes à gratter la muqueuse, l'une des rangées étant disposée selon une génératrice de la tige correspondante.

9. Procédé pour la réalisation d'un dispositif de prélèvement selon l'une des revendications précédentes, dans lequel on réalise la tige et les éléments en un même matériau, caractérisé en ce qu'on rapport les éléments de grattage (11,13) par moulage, en un matériau présentant une rigidité inférieure au matériau conformant la tige.

10. Procédé pour la réalisation d'un dispositif de prélèvement selon l'une des revendications 1 à 8, caractérisé en ce que l'on réalise par une opération de moulage bi-injection, l'ensemble de la tige et des éléments de grattage, en injectant simultanément dans un moule, d'une part un matériau de rigidité suffisante pour permettre l'utilisation du dispositif, et correspondant à la tige, et d'autre part, un matériau de rigidité inférieure, et correspondant aux éléments de grattage.
